# EUROPEAN PATENT APPLICATION

(11) **EP 4 840 441 A1**
(43) Date of publication of application: **18.11.2026**
(21) Application number: 25739126.8
(22) Date of filing: 10.01.2025
(51) Int. Cl.: A61K 9/00, A61K 47/24, A61K 47/26, A61K 47/22, A61K 47/44, A61P 3/04, A61P 29/00, A61P 1/08, A61P 19/02

(54) **SUSTAINED-RELEASE LIPID PRE-CONCENTRATE COMPRISING SORBITAN SATURATED FATTY ACID AND METHOD FOR PREPARING SAME**

(30) Priority: 12.01.2024 KR 20240005523
(71) Applicant: TIONLAB THERAPEUTICS CO., LTD., Gyeongsangnam-do 50969 (KR)
(72) Inventor: BAIK, Hye Jung, Seoul 06787 (KR); LIM, Duck Soo, Suwon-si Gyeonggi-do 16628 (KR)
(74) Representative: Schiweck Weinzierl Koch Patentanwälte Partnerschaft mbB
(86) International application number: PCT/KR2025/000606
(87) International publication number: WO 2025/150966

(57) **Abstract**

The present invention relates to a lipid pre-concentrate and a method for preparing same, wherein the lipid pre-concentrate uses a biodegradable lipid having low injection force and low toxicity to delay the release rate of a drug, thereby exhibiting sustained release so that the administered drug can be continuously released for a long period of time.

## Description

### [Technical Field]

The present invention relates to a lipid preconcentrate having low injection force and exhibiting sustained release by delaying the release rate of a drug using sorbitan saturated fatty acid ester, which is a biodegradable lipid having low toxicity, thereby enabling continuous release of the administered drug over a long period of time, and to a method for preparing the same.

### [Background Art]

A sustained-release formulation is a formulation capable of continuously releasing a pharmacologically active material through a single administration, thereby preventing side effects that may occur upon repeated administration and maintaining the effective concentration range of the pharmacologically active material for a certain period of time or longer.

A currently used representative sustained-release material that has biodegradability is poly(lactic-co-glycolic acid) (PLGA), which has been approved by the Food and Drug Administration (FDA). U.S. Patent No. 5,480,656 discloses that a PLGA biodegradable polymer is decomposed into lactic acid and glycolic acid after a certain period of time *in vivo*, thereby enabling continuous release of a pharmacologically active material. However, acidic materials, which are degradation products of PLGA, may cause inflammatory responses and decrease a cell proliferation rate (K. Athanasiou, G. G. Niederauer, and C. M. Agrawal, Biomaterials, 17, 93 (1996)). In addition, for sustained release, a drug should be encapsulated in PLGA solid particles of approximately 10 to 100 micrometers and then injected, but in this case, there is a problem in that pain or inflammation accompanies injection. Accordingly, there is a need for development of a new sustained release formulation capable of providing an effective concentration of a pharmacologically active material for a certain period of time or longer and improving patient compliance.

As a formulation capable of overcoming the drawbacks of biodegradable polymer-based formulations, WO 2005/117830 discloses a liquid depot formulation including at least one neutral diacyl lipid (e.g., diacyl glycerol such as glyceryl dioleate) and/or at least one tocopherol; at least one phospholipid; and at least one low-viscosity biocompatible organic solvent containing oxygen. However, formulations containing a neutral diacyl lipid such as glyceryl dioleate have a problem of low biodegradability, and since they are not biodegradable and are not biologically derived materials, they have limitations in biocompatibility and a high possibility of causing inflammation.

Japanese Patent No. 5681626 discloses that a sustained-release formulation including microspheres in which a sterol-containing lipid component and a PLGA polymer are combined and a phospholipid component can control the release rate. However, in the case of lipids, they do not exhibit a sufficient effect in suppressing initial release, and have a drawback in that their strong hydrophobicity causes poor characteristics of the microspheres. In addition, modification of the PLGA polymer forming the microspheres causes difficulties in obtaining approval for the use of the drug, and thus there are limitations in applying and practically using the corresponding composition in a depot formulation.

Therefore, there is a need in the art to develop a lipid formulation for sustained-release injections, as a long-acting drug injectable formulation, which can prevent an initial drug release phenomenon and has excellent biodegradability, biocompatibility, and injection force.

### [Related Art Documents]

1. U.S. Patent No. 5,480,656
2. International Patent Publication No. WO 2005/117830
3. Japanese Patent No. 5681626

### [Detailed Description of Invention]

### [Technical Problem]

The present invention is directed to providing a lipid preconcentrate that has low injection force and delays the release rate, thereby exhibiting sustained release so that the administered drug can be continuously released for a long period of time.

The present invention is also directed to providing a method of preparing a lipid preconcentrate exhibiting the above-described effect.

### [Technical Solution]

The present invention provides a lipid preconcentrate, which includes: a phospholipid; a sorbitan saturated fatty acid; and squalene, wherein
a liquid crystal is formed in an aqueous medium.

The present invention also provides a method of preparing the above-described lipid preconcentrate, which includes preparing a liquid solution by mixing a phospholipid; a sorbitan saturated fatty acid; and squalene.

### [Advantageous Effects]

The lipid preconcentrate of the present invention can form a liquid crystal upon exposure to an aqueous medium, enabling sustained drug release, and can maintain drug release for approximately one week to one month. In addition, the liquid crystal formed by the lipid preconcentrate exhibits sustained-release properties even under low-viscosity conditions, excellent viscoelasticity, and superior ease of use due to low injection force during injection.

### [Description of Drawings]

FIG. 1 is a set of images showing that lipid preconcentrates prepared in Preparation Example 1 are injected into a pH 7.4 phosphate buffer solution.
FIG. 2 is a set of images showing that lipid preconcentrates prepared in Preparation Example 2 are injected into a pH 7.4 phosphate buffer solution.
FIG. 3 is a set of images taken after a certain period of time following the administration of the lipid preconcentrates prepared in Preparation Example 2 to SD RAT.
FIG. 4 shows images of the physical appearance (left) and gel state (right) of the lipid preconcentrate prepared in Preparation Example 3-1.
FIG. 5 shows images of the physical appearance (left) and the gel state (right) of the lipid preconcentrate prepared in Preparation Example 3-13.
FIG. 6 shows the polarizing microscope (PLM) analysis result of the lipid preconcentrate prepared in Preparation Example 3-1.
FIG. 7 shows the Cryo-TEM analysis result of the lipid preconcentrate of Preparation Example 3-1.
FIG. 8 shows the animal PK evaluation result of the lipid preconcentrate prepared in Preparation Example 3-1 and the lipid preconcentrate prepared in Comparative Example.
FIG. 9 shows the bio-imaging analysis result of the lipid preconcentrate prepared in Preparation Example 3-1.

### [Best Mode]

The present invention relates to a lipid preconcentrate, which includes a phospholipid; a sorbitan saturated fatty acid; and squalene, wherein
a liquid crystal is formed in an aqueous medium.

Hereinafter, the present invention will be described in detail.

In the present invention, a preconcentrate corresponds to a sustained-release formulation capable of releasing a drug over an extended period of time, and more specifically, may be a sustained-release injectable formulation or oral formulation. The sustained-release formulation must gradually release the drug therein at a predetermined level over a certain period of time. In the present invention, the preconcentrate has the form of a pre-formulated lipid solution, and forms a non-lamellar phase, specifically, a non-lamellar liquid crystal (hereinafter referred to as a liquid crystal) during exposure to an aqueous medium such as water or a biological fluid. That is, the lipid preconcentrate of the present invention may form a long-lasting liquid crystal in the body. The liquid crystal may be used interchangeably with a gel or depot.

In the present invention, the lipid preconcentrate includes a phospholipid; a sorbitan saturated fatty acid; and squalene.

In the present invention, the phospholipid may serve as a liquid crystal-forming agent.

In one embodiment, the phospholipid may be at least one selected from the group consisting of lecithin, soybean phospholipid, egg phospholipid, phosphatidylcholine, phosphatidylethanolamine, phosphatidylserine, phosphatidic acid, phosphatidylglycerine, sphingomyelin, phosphatidylinositol, hydrated phospholipid, and lysophospholipid.

In one embodiment, the content of the phospholipid may be 30 to 60 wt%, or 30 to 45 wt% of lipid preconcentrate based on the total weight (100 wt%). When the content of the phospholipid is less than 30 wt% or more than 60 wt%, the *in vivo* injection of the lipid preconcentrate may significantly degrade the liquid crystal-forming ability and decrease the sustained release properties of a drug to be described below. As a result, there is a concern that the release control capability of at least one week intended by the present invention may not be achieved.

In the present invention, the sorbitan saturated fatty acid may serve as a liquid crystal-forming agent, such as a phospholipid.

In one embodiment, the sorbitan saturated fatty acid may be at least one selected from the group consisting of sorbitan caprylate, sorbitan laurate, sorbitan monolaurate, sorbitan myristate, sorbitan palmitate, sorbitan monopalmitate, sorbitanstearate, sorbitan monostearate, sorbitan tristearate, sorbitan isostearate, and sorbitan monomyristate.

The content of the sorbitan saturated fatty acid may be 5 to 45 wt%, 10 to 35 wt%, 15 to 30 wt%, or 25 to 30 wt% based on the total weight (100 wt%) of the lipid preconcentrate. Within this content range, the release control capability of at least one week can be secured.

In the present invention, the squalene may serve as a liquid crystal reinforcing agent. That is, the squalene may help strongly stiffen the internal structure of the liquid crystal, and may serve to delay a drug release rate.

In one embodiment, the content of the squalene may be 2.5 to 30 wt%, or 5 to 15 wt% based on the total weight (100 wt%) of the lipid preconcentrate. Within this content range, the internal structure of the liquid crystal may be strongly maintained.

The lipid preconcentrate of the present invention may serve as a sustained-release formulation capable of releasing a drug for a long period of time, and may be used in the form of a sustained-release injectable formulation. In the present invention, by using the lipid preconcentrate as a sustained-release injectable formulation, the drug contained in the formulation may be induced and adjusted to be gradually released at an intended level.

The lipid preconcentrate of the present invention may further include a solvent. The solvent may serve to improve the dissolution of a main component, or ease of injection. When the sustained-release, lipid preconcentrate including a solvent is injected into the body, the solvent may be removed by substitution or dilution in a biological fluid during the generation of a liquid crystal.

In one embodiment, the type of solvent is not particularly limited, and any solvent that can be introduced into the body in the form of an injection may be used. For example, the solvent may be at least one selected from the group consisting of water, ethanol, isopropanol, propyleneglycol, N-methylpyrrolidone, glycerol, benzylbenzoate, acetic acid, and benzylalcohol.

In one embodiment, the content of the solvent may be 5 to 25 wt%, or 10 to 20 wt% based on the total weight (100 wt%) of the lipid preconcentrate.

In one embodiment, when water is used as a solvent, it may be used in the form of a mixture solution with a second solvent. The second solvent may be the above-mentioned solvent. Here, the water content may be 5 wt% or less, or 3 wt% or less, and the content of the second solvent may be 5 to 20 wt%. The above-mentioned content of water does not affect the formation of a liquid crystal during the preparation and storage of the lipid preconcentrate.

The lipid preconcentrate of the present invention may further include a stabilizing agent. The stabilizing agent may help stably maintain the internal structure of the liquid crystal, and enable preparation of a strong liquid crystal.

In one embodiment, the type of stabilizing agent is not particularly limited, and may be at least one selected from the group consisting of tocopherol, tocopherol acetate, and cholesterol.

The lipid preconcentrate of the present invention may further include an antioxidant. The antioxidant may be used to improve the stability of the lipid preconcentrate and drug.

In one embodiment, the type of antioxidant is not particularly limited, and may be at least one selected from the group consisting of sodium ascorbate, (ViC_Na), ascorbic palmitate (ViC_palmitate), butylated hydroxyanisole (BHA), butylated hydroxyltoluene (BHT), methionine, monothioglycerol, sodium thiosulfate, and sodium metabisulfite.

The lipid preconcentrate of the present invention may further include a surfactant. The surfactant may serve to improve the dissolution of a main component.

In one embodiment, the surfactant may be at least one selected from the group consisting of polysorbate (tween), Cremophor, Solutol, Brij, Triton, sugar fatty acid esters (e.g., sucrose oleate, sucrose palmitate, and/or sucrose laurate), and surfactants with a polyoxyethylene head group.

The lipid preconcentrate of the present invention may further include a drug.

In one embodiment, the drug may be selected from the group consisting of an anti-obesity agent, an analgesic, an osteoarthritis therapeutic agent, and an agent for treating alopecia or benign prostatic hyperplasia.

In one embodiment, the anti-obesity agent may be semaglutide, tirzepatide, or liraglutide.

In one embodiment, the analgesic may be cerecoxib or meloxicam.

In one embodiment, the osteoarthritis therapeutic agent may be betamethasone, dexamethasone, methylprednisolone, prednisolone, or triamcinolone.

In one embodiment, the agent for treating alopecia or benign prostatic hyperplasia may be dutasteride or finasteride.

In the present invention, the name of the lipid preconcentrate may vary depending on the type of drug. For example, when semaglutide is used as a drug, the lipid preconcentrate prepared may be named a semaglutide lipid preconcentrate.

The lipid preconcentrate of the present invention may be used as a pharmaceutical composition according to the effect, characteristics, and use of the drug. The pharmaceutical composition may delay the release of the drug to exhibit continuous effectiveness.

The lipid preconcentrate of the present invention may not include diacyl glycerol and/or sorbitan unsaturated fatty acid ester. The diacyl glycerol may be glyceryl dipalmitate, glyceryl phytanoate, glyceryl palmitoleate, glyceryl distearate, glyceryl dioleate, glyceryl dielaidiate, and glyceryl dilinoleate, and the sorbitan unsaturated fatty acid ester may be sorbitan monooleate, sorbitan monolinoleate, sorbitan monopalmitoleate, sorbitan monomyristoleate, sorbitan sesquioleate, sorbitan sesquilinoleate, sorbitan sesquipalmitoleate, sorbitan sesquimyristoleate, sorbitan dioleate, sorbitan dilinoleate, sorbitan dipalmitoleate, and sorbitan dimyristoleate.

In addition, the lipid preconcentrate of the present invention may not include 14 to 20 unsaturated fatty acids.

Specifically, the lipid preconcentrate of the present invention may be a pharmaceutical composition that does not include at least one component selected from diacyl glycerol, sorbitan unsaturated fatty acid esters, and unsaturated fatty acids having 14 to 20 carbon atoms.

The lipid preconcentrate according to the present invention forms a liquid crystal in an aqueous medium.

In one embodiment, the aqueous medium may be water, a biological fluid, or a phosphate buffer solution (PBS).

The lipid preconcentrate according to the present invention may have an injection force of 1 to 30 N.

Injection force refers to the injection force at an injection rate that is comfortable for the patient. "Comfortable for the patient" is used to define an injection rate that does not cause injury or excessive pain to the patient during injection. As used herein, "comfort" includes not only the comfort of the patient, but also the comfort or ability of a physician or medical professional in administering the composition by injection. In the present invention, injection force may be measured using an INTRONS 5569 instrument under the conditions of an experimental example to be described below. In general, a low injection force allows easy control without any pain during administration of the composition.

In the present invention, the injection force of the lipid preconcentrate may be 1 to 30 N, or 1 to 13 N.

In addition, the present invention relates to a method of preparing the above-described lipid preconcentrate. The types and contents of components used in the method of preparing the lipid preconcentrate are the same as described for the lipid preconcentrate.

The method of preparing the lipid preconcentrate of the present invention may include preparing a lipid solution by mixing a phospholipid; a sorbitan saturated fatty acid; and squalene (hereinafter, lipid solution preparation). In the preparation method, the lipid solution may be used as a lipid preconcentrate.

In the lipid solution preparation of the present invention, the lipid solution may further include a solvent. In this case, the lipid solution may be prepared by mixing and dissolving the phospholipid; sorbitan saturated fatty acid; and squalene in a solvent.

In one embodiment, the lipid solution may further include a drug.

The drug may be at least one selected from the group consisting of an anti-obesity agent, an analgesic, an osteoarthritis therapeutic agent, and a therapeutic agent for alopecia and benign prostatic hyperplasia.

In one embodiment, the lipid solution may further include a stabilizing agent, an antioxidant, and/or a surfactant.

In the present invention, the lipid solution may be used being injected into a container or syringe.

In addition, the lipid preconcentrate of the present invention may be prepared by preparing a lipid solution by mixing a phospholipid; a sorbitan saturated fatty acid; and squalene (hereinafter, lipid solution preparation);
preparing a drug solution containing a drug (hereinafter, drug solution preparation); and
mixing the lipid solution with the drug solution (hereinafter, mixing). In the preparation method, the mixture solution prepared through the mixing in the preparation method may be used as a lipid preconcentrate.

In the lipid solution preparation of the present invention, the lipid solution may further include a solvent. In this case, the lipid solution may be prepared by mixing and dissolving the phospholipid; the sorbitan saturated fatty acid; and the squalene in a solvent.

In one embodiment, the lipid solution may further include a stabilizing agent, an antioxidant, and/or a surfactant.

In the present invention, the lipid solution may be used by being injected into a container or syringe.

In the drug solution preparation of the present invention, the drug solution may be prepared by mixing and dissolving the drug in a solvent.

In one embodiment, the drug solution may be used by being injected into a container or syringe.

The mixing of the present invention may be performed by the method of mixing the lipid solution injected into a container or syringe with the drug solution injected into a container or syringe.

In one embodiment, the mixing may be performed by connecting at least two syringes to interchange the solutions in the syringes. The interchange may be repeated twice or more, and particularly, twice to 100 times, 10 to 80 times, 30 to 70 times, 40 to 60 times, or 50 times.

In addition, the present invention relates to a sustained-release injectable pharmaceutical composition including the above-described lipid preconcentrate.

In one embodiment, the sustained-release injectable pharmaceutical composition may be used for subcutaneous or intramuscular injection.

Moreover, the present invention relates to an oral pharmaceutical composition including the above-described sustained-release lipid preconcentrate.

The present invention may have various modifications and various examples, and thus specific examples are illustrated in the drawings and described in detail in the detailed description. However, it should be understood that the present invention is not limited to specific embodiments, and includes all modifications, equivalents or alternatives within the spirit and technical scope of the present invention.

### [Modes of the Invention]

Hereinafter, the present invention will be described in detail with reference to examples. However, the following preparation examples and experimental examples specifically illustrate the present invention, and the scope of the present invention is not limited thereto.

### Examples

### Preparation Example 1. Preparation of lipid preconcentrate using sorbitan saturated fatty acid

A lipid preconcentrate was prepared with the components and contents (g) listed in Table 1 below.

Specifically, after the components listed in Table 1 below were added to a glass vial, the mixture was stirred and mixed using a magnetic stirrer at room temperature to 60°C. The mixture was then allowed to stand at room temperature for about 3 hours to prepare the lipid preconcentrate.

**[Table 1]**

| | Preparatio n Example 1-1 | Preparatio n Example 1-2 | Preparatio n Example 1-3 | Preparatio n Example 1-4 | Preparatio n Example 1-5 | Preparatio n Example 1-6 | Preparatio n Example 1-7 | Preparatio n Example 1-8 |
|---|---|---|---|---|---|---|---|---|
| Phosphatidyl choline | 35 | 35 | 35 | 35 | 35 | 35 | 35 | 35 |
| Sorbitan laurate(conten t, wt%) | 0 (0 %) | 5 (7.7%) | 10 (14.3%) | 15 (20.0%) | 20 (25.0%) | 30 (33.3%) | 40 (40.0%) | 30 |
| Squalene | 10 | 10 | 10 | 10 | 10 | 10 | 10 | 0 |
| Ethanol | 15 | 15 | 15 | 15 | 15 | 15 | 15 | 15 |

### Experimental Example 1. Confirmation of gel state from aqueous phase of lipid preconcentrate

The lipid preconcentrate prepared in Preparation Example 1 was injected into 5 mL of a pH 7.4 phosphate buffer solution (PBS) using a pipette.

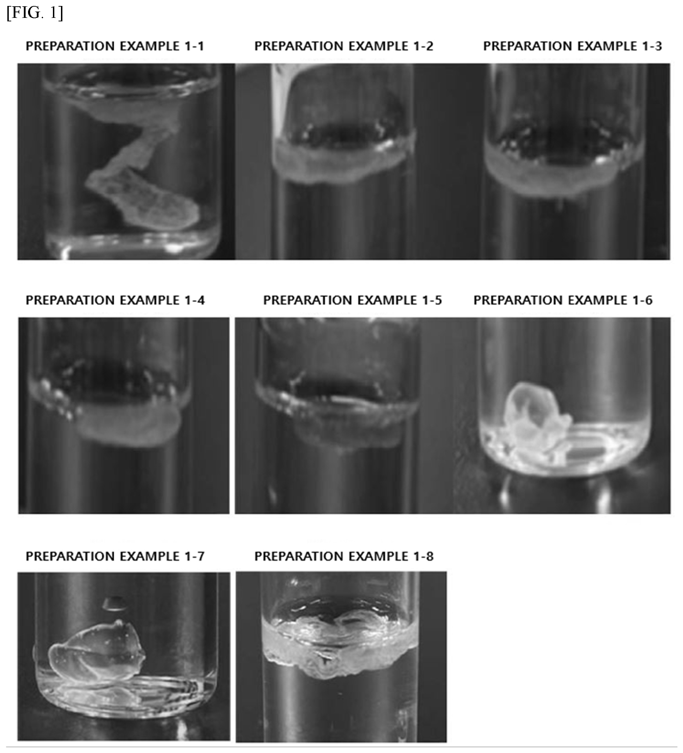

FIG. 1 is a set of images showing that lipid preconcentrates prepared in Preparation Example 1 are injected into a pH 7.4 phosphate buffer solution. Referring to FIG. 1, it can be confirmed that, as the content of sorbitan laurate, which is a sorbitan saturated fatty acid, increases, gel formation in the aqueous phase becomes stronger.

In addition, it can be confirmed that no gel was formed when sorbitan laurate or squalene was not included.

Accordingly, it can be confirmed that the lipid preconcentrate of the present invention exhibited a liquid state under normal conditions and was converted into a gel state upon contact with an aqueous medium.

### Preparation Example 2. Preparation of lipid preconcentrate using liquid crystal forming agent

A lipid preconcentrate was prepared with the components and contents (g) listed in Table 2 below.

Specifically, after the components in Table 2 below were added to a glass vial, the mixture was stirred and mixed using a magnetic stirrer at room temperature to 60 °C. The mixture was then allowed to stand at room temperature for about 3 hours to prepare the lipid preconcentrate.

In table 2, oleic acid is an unsaturated fatty acid, sorbitan monooleate and sorbitan sesquioleate are sorbitan unsaturated fatty acids, stearic acid and myristic acid are saturated fatty acids, and glyceryl dioleate is a glyceryl unsaturated fatty acid.

**[Table 2]**

| | Preparati on Example 1-6 | Preparati on Example 2-1 | Preparati on Example 2-2 | Comparat ive Preparatio n Example 2-1 | Comparat ive Preparatio n Example 2-2 | Comparat ive Preparatio n Example 2-3 | Comparat ive Preparatio n Example 2-4 | Comparat ive Preparatio n Example 2-5 | Comparat ive Preparatio n Example 2-6 |
|---|---|---|---|---|---|---|---|---|---|
| Phosphati dyl choline | 35 | 35 | 35 | 35 | 35 | 35 | 35 | 35 | 35 |
| Sorbitan laurate | 30 | - | - | - | - | - | - | - | - |
| Sorbitan | - | 30 | - | - | - | - | - | - | - |
| stearate | | | | | | | | | |
| Sorbitan caprylate | | - | 30 | - | - | - | - | - | - |
| Oleic acid | - | | - | 30 | - | - | - | - | - |
| Sorbitan monoolea te | - | - | - | - | 30 | - | - | - | - |
| Sorbitan sesquiole ate | - | - | - | - | - | 30 | - | - | - |
| Strearic acid | - | - | - | - | - | - | 30 | - | - |
| Myristic acid | - | - | - | - | - | - | - | 30 | - |
| Glyceryl dioleate | - | - | - | - | - | - | - | - | 30 |
| Squalene | 10 | 10 | 10 | 10 | 10 | 10 | 10 | 10 | 10 |
| Ethanol | 15 | 15 | 15 | 15 | 15 | 15 | 15 | 15 | 15 |

### Experimental Example 2. Confirmation of gel state from aqueous phase of lipid preconcentrate

The lipid preconcentrate prepared in Preparation Example 2 was injected into 5 mL of a pH 7.4 phosphate buffer solution (PBS) using a pipette.

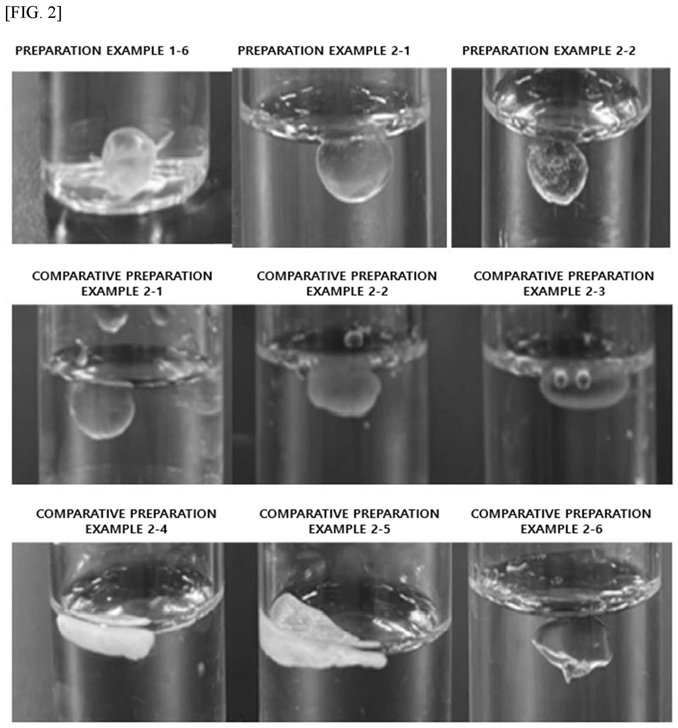

FIG. 2 is a set of images showing that lipid preconcentrates prepared in Preparation Example 2 are injected into a pH 7.4 phosphate buffer solution.

As shown in FIG. 2, it can be confirmed that stearic acid (Comparative Preparation Example 2-4) or myristic acid (Comparative Preparation Example 2-5), which are saturated fatty acids, formed very weak gels, whereas sorbitan laurate (Preparation Example 1-6), sorbitan stearate (Preparation Example 2-1), and sorbitan caprylate (Preparation Example 2-2), which are sorbitan saturated fatty acids, formed strong liquid crystals.

It can be confirmed that when sorbitan sesquioleate (Comparative Preparation Example 2-3), which is a sorbitan unsaturated fatty acid, was applied, a relatively weak gel was formed, exhibiting spreading on the surface of the aqueous phase over time.

### Experimental Example 3. Experiment for confirming injection force of lipid preconcentrate

The lipid preconcentrate was filled into a syringe, and the effect on injection force was evaluated.

Specifically, injection force was evaluated based on the load (N) measured when a 23-gauge syringe mounted on an INTRON 5569 instrument was compressed at a rate of 100 mm/min.

Since injection needles commonly used for general liquid injections do not cause severe pain and are convenient to use when having a gauge of 23 or higher, needles of 23 gauge or higher are used. Particularly, when the load is 10 N or less, a very small needle of 26 gauge or higher may be used, thereby providing low pain to a patient and achieving low injection force even with a small force.

Table 3 below shows the measurement result for the injection force of the lipid preconcentrate prepared in Preparation Example 2.

**[Table 3]**

| | Preparation Example 1-6 | Comparative Preparation Example 2-1 | Comparative Preparation Example 2-2 | Comparative Preparation Example 2-3 | Comparative Preparation Example 2-4 | Comparative Preparation Example 2-5 | Comparative Preparation Example 2-6 |
|---|---|---|---|---|---|---|---|
| Injection force (N) | 9 N | 15 N | 32 N | 35 N | 9 N | 9 N | 41 N |

As shown in Table 3, it can be confirmed that, compared to the formulation containing the sorbitan saturated fatty acid (Preparation Example 1-6) of the present invention, the formulation containing the sorbitan unsaturated fatty acids (Comparative Preparation Example 2-2, 2-3), the glyceryl unsaturated fatty acid (Comparative Preparation Example 2-6), or the monosaturated fatty acid (Comparative Preparation Example 2-1) could be injected only when a strong force was applied during injection. Although the formulations containing the saturated fatty acids (Comparative Preparation Examples 2-4 and 2-5) exhibited low injection force, they were not suitable as components of the lipid preconcentrate due to their low gel-forming ability in the aqueous phase.

That is, it can be confirmed that the sorbitan saturated fatty acid exhibited an excellent gel-forming ability in the aqueous phase despite its low injection force.

### Experimental Example 4. Evaluation of local site irritation after subcutaneous administration of lipid preconcentrate in SD rat

The lipid preconcentrate prepared in Preparation Example 2 was charged in a syringe and then subcutaneously administered to the dorsal area of an SD rat. After a predetermined period of time, skin irradiation at the local administration site was evaluated.

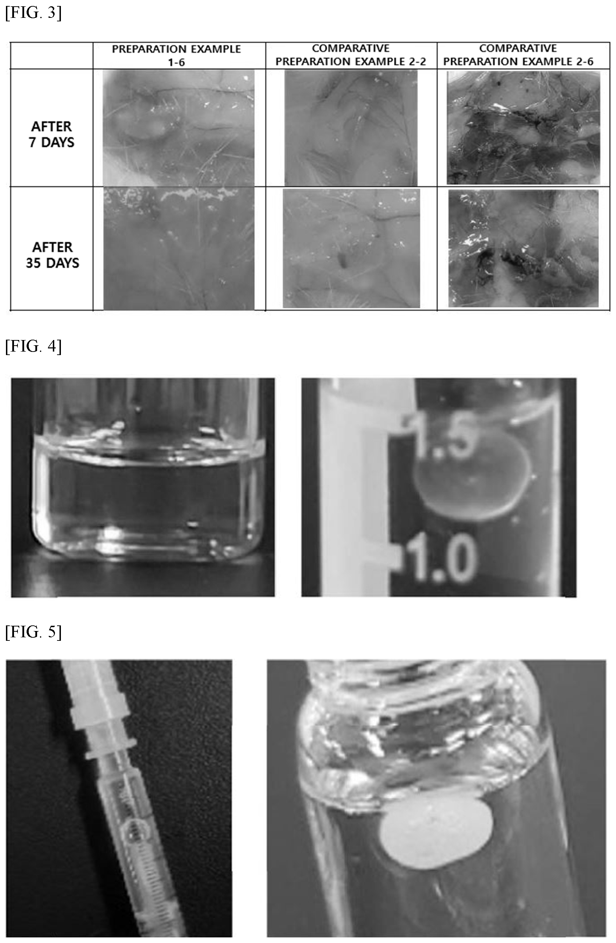

FIG. 3 is a set of images taken after a certain period of time (7 days) following the administration of the lipid preconcentrates prepared in Preparation Example 2 to SD RAT.

As shown in FIG. 3, it can be confirmed that Comparative Preparation Example 2-6 using glyceryl dioleate (GDO), which is a glyceryl unsaturated fatty acid, exhibited inflammatory responses caused by pus formation and excessive angiogenesis. In contrast, it can be confirmed that when the sorbitan saturated fatty acid according to the present invention was used, little to no inflammatory response was observed.

In addition, when the glyceryl unsaturated fatty acid was applied, there is a problem in that, even after 35 days, the formulation is not completely biodegraded and thus a portion thereof may remain. When the sorbitan unsaturated fatty acid is applied (Comparative Preparation Example 2-2), the formulation is not completely biodegraded, and a small amount thereof may remain. When the sorbitan saturated fatty acid according to the present invention is applied, the formulation is mostly biodegraded, thereby ensuring its stability.

### Preparation Examples 3 and 4. Preparation of lipid preconcentrate containing semaglutide and tirzepatide drugs

According to the components and contents (g) in Tables 4 to 6 below, lipid preconcentrates containing drugs were prepared.

Specifically, in the cases of Comparative Preparation Examples 3-1 and 3-2, Preparation Examples 3-1 to 3-12, Comparative Preparation Example 4-1, and Preparation Examples 4-1 to 4-4, the components (content: g) listed in Table 4 below were sequentially added to a glass vial, and then stirred and mixed using a magnetic stirrer at room temperature to 60 °C. The mixture was left at room temperature for approximately 3 hours, thereby preparing a lipid preconcentrate.

In the cases of Preparation Example 3-13 and Preparation Example 3-14, among the compositions listed in Table 5 below, all components except semaglutide corresponding to the drug were mixed and dissolved to prepare a solution (lipid solution preparation), and semaglutide was separately dissolved in water for injection to prepare a solution (drug solution preparation). Thereafter, the lipid solution and the drug solution were each charged into a syringe, and the syringes were connected such that the drug contained therein was alternately transferred back and forth between the two syringes 50 times or more to mix the solutions and thereby prepare a lipid preconcentrate.

**[Table 4]**

| | Comparativ e | Comparativ e | Preparatio n Example | Preparatio n Example | Preparatio n Example | Preparatio n Example | Preparatio n Example | Preparatio n Example |
|---|---|---|---|---|---|---|---|---|
| | Preparation Example 3-1 | Preparation Example 3-2 | 3-1 | 3-2 | 3-3 | 3-4 | 3-5 | 3-6 |
| Semaglutid e | 5 | 5 | 5 | 5 | 5 | 5 | 5 | 5 |
| Water | - | - | - | - | - | - | 2.5 | - |
| Acetic acid | 5 | 5 | 5 | 5 | 5 | 5 | 5 | 5 |
| Phosphatidy 1 choline | 35 | 35 | 35 | 40 | 50 | 60 | 35 | 35 |
| Sorbitan laurate | - | - | 25 | 20 | 20 | 15 | 22.5 | 25 |
| Sorbitan monooleate | 25 | | | | | | | |
| Glyceryl dioleate | | 25 | | | | | | |
| Ascorbic palmitate | - | - | - | - | - | - | - | - |
| Tocopherol acetate | - | - | - | - | - | - | - | 5 |
| Cholesterol | 5 | 5 | 5 | 5 | - | - | 5 | - |
| Squalene | 10 | 10 | 10 | 10 | 10 | 10 | 10 | 10 |
| Ethanol | 15 | 15 | 15 | 15 | 10 | 10 | 15 | 15 |
| Tween 80 | - | - | - | - | - | - | - | - |
| Result of gel formation | Gel formed | Gel formed | Gel formed | Gel formed | Gel formed | Gel formed | Gel formed | Gel formed |

**[Table 5]**

| | Preparatio n Example 3-7 | Preparatio n Example 3-8 | Preparatio n Example 3-9 | Preparatio n Example 3-10 | Preparatio n Example 3-11 | Preparatio n Example 3-12 | Preparatio n Example 3-13 | Preparatio n Example 3-14 |
|---|---|---|---|---|---|---|---|---|
| Semaglutide | 5 | 5 | 5 | 5 | 5 | 5 | 2 | 2 |
| Water | - | - | 5 | - | - | - | 3 | 3 |
| Acetic acid | 5 | 5 | - | 5 | 5 | 5 | - | - |
| Phosphatidy | 40 | 35 | 35 | 35 | 25 | 30 | 40 | 40 |
| 1 choline | | | | | | | | |
| Sorbitan laurate | 30 | 25 | 25 | 20 | 45 | 15 | 25 | 25 |
| Sorbitan monooleate | | | | | | | | |
| Glyceryl dioleate | | | | | | | | |
| Ascorbic palmitate | - | - | - | 5 | - | - | - | - |
| Tocopherol acetate | - | 5 | - | 5 | 5 | 5 | 3 | 3 |
| Cholesterol | 2.5 | 5 | 5 | 5 | 5 | 5 | - | - |
| Squalene | 2.5 | 5 | 10 | 10 | 20 | 30 | 12 | 12 |
| Ethanol | 15 | 15 | 15 | 15 | 5 | 10 | 15 | 15 |
| Tween 80 | - | - | 0.5 | - | - | - | - | - |
| Result of gel formation | Gel formed | Gel formed | Gel formed | Gel formed | Weak gel formed | Weak gel formed | Gel formed | Gel formed |

**[Table 6]**

| | Preparation Example 4-1 | Preparation Example 4-2 | Preparation Example 4-3 | Preparation Example 4-4 | Comparative Preparation Example 4-1 |
|---|---|---|---|---|---|
| Tirzepatide | 1 | 1 | 1 | 1 | 1 |
| Phosphatidyl choline | 35 | 35 | 40 | 40 | 35 |
| Sorbitan laurate | 30 | 25 | 20 | 20 | - |
| Ascorbic palmitate | - | - | - | 5 | - |
| Tocopherol acetate | 5 | - | 5 | - | - |
| Squalene | 5 | 15 | 15 | 15 | 5 |
| Cholesterol | 5 | 5 | - | - | 5 |
| Ethanol | 10 | 10 | 10 | 10 | 10 |
| DMSO | 9 | 9 | 9 | 9 | 9 |
| Results of gel formation | Gel formed | Gel formed | Gel formed | Gel formed | No gel formation |

The lipid preconcentrate was injected into 5 mL of a pH 7.4 phosphate buffer solution using a pipette.

As a result of confirming gel formation, it can be confirmed that the lipid preconcentrate of Preparation Example 3-1 exhibited a liquid state under normal conditions and was converted into a gel state upon contact with the pH 7.4 phosphate buffer solution (FIG. 4).

In addition, like Preparation Example 3-13, even in the case of the method in which a syringe containing the drug dissolved in water for injection and a syringe containing the lipid solution were connected, alternately mixed and then administered, it can be confirmed that the two solutions were well mixed to exhibit a transparent liquid state and were converted into a gel state in the pH 7.4 phosphate buffer solution (FIG. 5).

### Experimental Example 5. Confirmation of liquid crystal structure of semaglutide lipid preconcentrate

A lipid preconcentrate was applied onto a slide glass, and after spraying a pH 7.4 phosphate buffer solution onto the lipid preconcentrate, the resulting mixture was allowed to stand for approximately 1 minute. After gently removing excess moisture, a cover glass was placed and fixed thereon, followed by observation using polarized light microscopy (PLM, NICON/LV100Pol, 5x magnification).

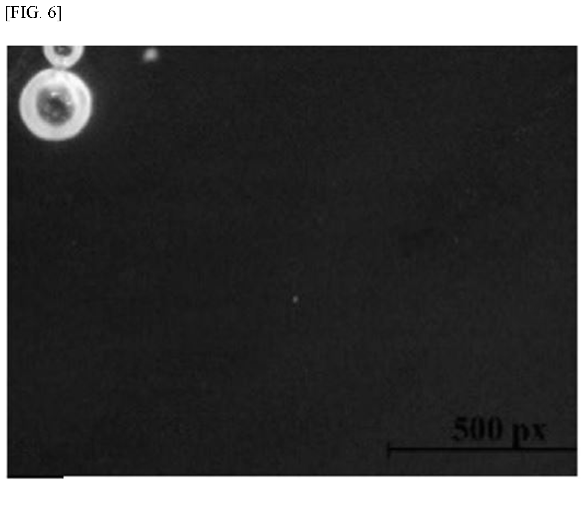

FIG. 6 shows polarized light microscopy images obtained after spraying the pH 7.4 phosphate buffer solution onto the semaglutide lipid preconcentrate prepared in Preparation Example 3-1. The images appear black and dark, which is characteristic of a cubic structure among non-lamellar structures.

In addition, the structure of the lipid preconcentrate was confirmed by Cryo-TEM.

Specifically, after forming a gel in the pH phosphate buffer solution, the gel was dispersed by sonication, and then the resulting sample was loaded on a mesh grid. Subsequently, it was frozen in ethane at -170 °C using liquid nitrogen. After fixing the grid onto a cassette in liquid nitrogen, the sample was observed at 200 kV using Cryo-TEM (Thermo Scientific^{™}, Glacios 2).

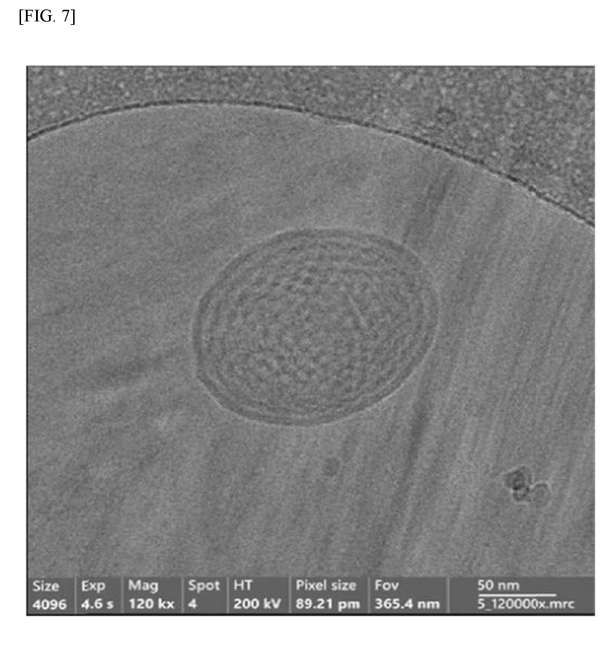

FIG. 7 shows the Cryo-TEM analysis result of the lipid preconcentrate of Preparation Example 3-1.

As shown in FIG. 7, a lattice structure is observed within the particles, confirming a characteristic feature of a non-lamellar structure.

### Experimental Example 6. Confirmation of sustained release of semaglutide and tirzepatide lipid preconcentrates in release tests

For the long-term release test (*in vitro* dissolution test) of the lipid preconcentrate, 5 mL of a pH 7.4 phosphate buffer solution containing 0.05% Tween 80 was added to a 6 cc glass vial, and the lipid preconcentrates of Comparative Preparation Example 3-1, Comparative Preparation Example 3-2, and Preparation Example 3-1 were injected thereinto using a pipette to form gels. The test was conducted for 28 days at a rotation speed of 30 rpm and a temperature of 37 °C using a rotator (PTR-35, lab23009). The collected samples were analyzed by HPLC under the following conditions.

### <HPLC conditions>

Column: Aegispak C18-L (4.6 x 250 mm, 3.0 µm) or a column having equivalent specifications
Mobile phase: A - a solution prepared by mixing 700 mL of a 10 mM phosphate buffer solution and 300 mL of acetonitrile, followed by adjustment to pH 2.0 with phosphoric acid
B - a solution prepared by mixing 200 mL of a 10 mM phosphate buffer solution and 800 mL of acetonitrile, followed by adjustment to pH 2.0 with phosphoric acid
Gradient condition mobile phase A(%): B(%)

**[Table 7]**

| Time (min) | MP A (%) | MP B (%) |
|---|---|---|
| 0 | 90 | 10 |
| 1 | 90 | 10 |
| 10 | 50 | 50 |
| 11 | 0 | 100 |
| 20 | 0 | 100 |
| 21 | 90 | 10 |
| 30 | 90 | 10 |

Flow rate: 1.2 mL/min, Temperature: 50 °C
Injection amount: 20 µL
Wavelength: 200 nm

The results of the release test of the semaglutide lipid preconcentrate are shown in Table 8 below.

**[Table 8]**

| | Long-term release, day 1 (%) | Long-term release, day 14 (%) | Long-term release, day 21 (%) | Long-term release, day 28 (%) | Long-term release, day 35 (%) |
|---|---|---|---|---|---|
| Comparative Preparation Example 3-1 | 5.5 | 76.4 | 100.0 | 100.0 | 100.0 |
| Comparative Preparation Example 3-2 | 4.3 | 26.3 | 27.6 | 41.5 | 71.5 |
| Preparation Example 3-1 | 11.5 | 58.1 | 75.5 | 87.5 | 100.0 |

As shown in Table 8, in the case of Comparative Preparation Example 3-1, it can be confirmed that 100% release was rapidly achieved on day 21, and in the case of Comparative Preparation Example 3-2, it can be confirmed that the formulation exhibited a release of 30% or less up to 3 weeks and still showed a low release of 50% or less even on day 38, indicating very slow drug release.

In contrast, it can be confirmed that Preparation Example 3-1 required 28 days for the release rate of 85% or higher, confirming more excellent and stable sustained-release properties over one month.

In addition, the results of the release test for the tirzepatide lipid preconcentrate are shown in Table 9 below.

**[Table 9]**

| | Long-term release, day 1 (%) | Long-term release, day 14 (%) | Long-term release, day 21 (%) | Long-term release, day 28 (%) | Long-term release, day 35 (%) |
|---|---|---|---|---|---|
| Preparation Example 4-1 | 8.5 | 48.1 | 65.5 | 89.5 | 100.0 |
| Comparative Preparation Example 4-1 | 5.5 | 100.0 | 100.0 | 100.0 | 100.0 |

As shown in Table 9, in the case of Comparative Preparation Example 4-1, it can be confirmed that the release rate of 100% was rapidly achieved on day 14. In contrast, in the case of Preparation Example 4-1, the release rate of 85% or higher was achieved up to 28 days, confirming more excellent stable sustained-release over one month.

### Experimental Example 7. Confirmation of sustained release properties of semaglutide lipid pre-concentrate formation in animal PK test

The sustained-release properties of the lipid preconcentrate were evaluated by conducting an animal PK test to confirm the drug release behavior.

The lipid preconcentrate of Preparation Example 3-1 was used, and Ozempic^{®} from Novo Nordisk was used as control drug. The drugs were subcutaneously injected into the backs of four 7- to 8-week-old male SD rats having an average body weight of 300 g. Here, the control drug was administered at 2.5 mpk, and the test drug was administered at 10 mpk. After administration, blood samples were collected at 0, 1, 3, 6, 12, 24, 48, 96, 168, 336, 576, and 672 hours, and plasma separation was performed at 12,000 rpm for approximately 2 minutes at 4 °C. The plasma concentrations in the SD rats were analyzed using LC-MS/MS, and pharmacokinetic analysis was performed by a non-compartmental method using Phoenix^{®} WinNonlin^{®} software (Ver. 8.3, Certara). The analysis conditions were as follows.

### <LC-MS/MS conditions>

Column: ACE C18 (2.1 x 50 mm, 3.0 µm), ACE
Mobile phase: A - 10 mM ammonium acetate buffer
B - acetonitrile containing 0.1% formic acid
Gradient condition mobile phase A(%): B(%)
Temperature: 40 °C

**[Table 10]**

| Time (min) | Flow (ml/min) | A | B |
|---|---|---|---|
| 0 | 0.4 | 55 | 45 |
| 1.5 | 0.4 | 5 | 95 |
| 2.5 | 0.4 | 5 | 95 |
| 2.51 | 0.4 | 55 | 45 |
| 4 | 0.4 | 55 | 45 |

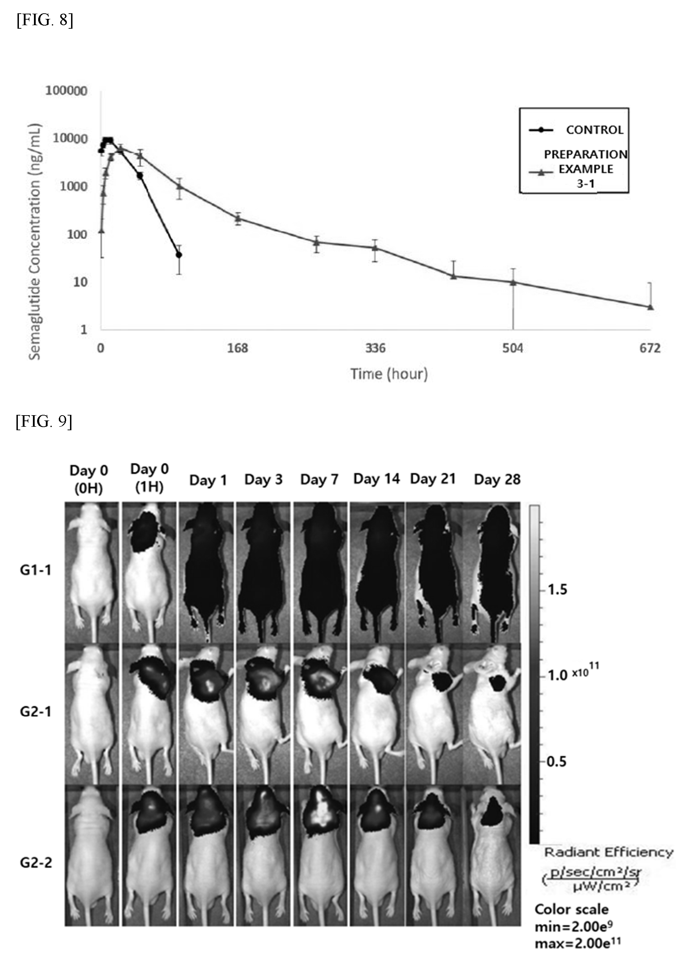

FIG. 8 shows the change in blood concentration of semaglutide.

As shown in FIG. 8, compared with the control drug, it can be confirmed that the lipid preconcentrate of Preparation Example 3-1 maintained the blood concentration for 28 days.

### Experimental Example 8. Bioimaging test for semaglutide lipid preconcentrate in animals

A fluorescent material was loaded in the lipid preconcentrate to perform a bio-imaging test on a mouse.

A lipid preconcentrate was prepared using 30 µg of DilC Dye (Invitrogen) instead of a drug in Preparation Example 3-1. After dissolving the lipid preconcentrate, approximately 200 µL of the prepared sample was subcutaneously administered to BALB/c nude mice (male, 5-6 weeks old). Images were acquired using a bio-imaging system (Lumina X5, PerkinElmer) before administration and at 1, 24, 48, 72, 168, 504, and 672 hours after administration. During imaging, inhalation anesthesia was induced using isoflurane, and imaging was performed at the dye wavelengths (ex 620 nm/em 670 nm).

FIG. 9 shows changes in the fluorescence intensity of the fluorescent material in the formulation loaded with the fluorescent material administered to mice.

Specifically, G1 refers to a case in which DilC Dye (Invitrogen) dissolved in a solvent at a concentration of 0.15 µL was subcutaneously administered in an amount of 200 µL, and G2-1 and G2-2 refer to cases in which the same DilC Dye at a concentration of 0.15 µL was dissolved in the lipid preconcentrate and subcutaneously administered.

As shown in the drawings, in the case of G1, the fluorescence was observed at an overall low intensity as the fluorescent material rapidly spread throughout the body and gradually disappeared, whereas in the cases of G2-1 and G2-2, fluorescence from the fluorescent material was observed to be localized at the administration site of the formulation loaded with the fluorescent material. In addition, it was confirmed that the fluorescent material emitted more intense fluorescence on day 7 than at the initial stage. This appears to have resulted from the fluorescent material being released weakly at the initial stage and then being gradually released over time.

Accordingly, compared to G1, the G2-1 and G2-2 formulations serve to retain the drug at the local administration site while simultaneously releasing the drug slowly following local administration, and such an effect demonstrates, through bio-imaging, that the formulations exhibit sustained-release properties compared to a conventional liquid formulation. In view of these results, it was confirmed that the drug loaded in the lipid preconcentrate of the present invention can be slowly released *in vivo*.

### Preparation Example 5. Preparation of lipid preconcentrate including analgesic and osteoarthritis therapeutic agents

Celecoxib and meloxicam were used as analgesic agents, and dexamethasone and triamcinolone were used as osteoarthritis therapeutic agents.

After the components (amount: g) shown in Table 11 below were sequentially added to a glass vial, the mixture was stirred and mixed using a magnetic stirrer at room temperature to 60 °C. The mixture was then allowed to stand at room temperature for approximately 3 hours to prepare a lipid preconcentrate.

**[Table 11]**

| | Preparation Example 5-1 | Comparative Preparation Example 5-1 | Preparation Example 5-2 | Comparative Preparation Example 5-2 | Preparation Example 5-3 | Comparative Preparation Example 5-3 | Preparation Example 5-4 | Comparative Preparation Example 5-4 |
|---|---|---|---|---|---|---|---|---|
| Cerecoxib | 5 | 5 | - | - | - | - | - | |
| Meloxicam | - | - | 5 | 5 | - | - | - | |
| Dexamethaso ne | - | - | - | - | 5 | 5 | - | |
| Triamcinolon e | - | - | - | - | - | - | 5 | 5 |
| Phosphatidyl choline | 35 | 35 | 45 | 45 | 35 | 35 | 35 | 35 |
| Sorbitan laurate | 20 | - | 15 | - | 15 | - | 15 | - |
| Ascorbic palmitate | - | - | - | - | - | - | - | - |
| Tocopherol acetate | - | - | - | - | - | - | - | - |
| Squalene | 15 | 15 | 15 | 15 | 15 | 15 | 15 | 15 |
| Cholesterol | 5 | 5 | 5 | 5 | 5 | 5 | 5 | 5 |
| Ethanol | 10 | 10 | 10 | 10 | 10 | 10 | 10 | 10 |
| DMSO | 10 | 5 | 5 | 5 | 15 | 15 | 15 | 15 |
| Result of gel formation | Gel formed | No gel formation | Gel formed | No gel formation | Gel formed | No gel formation | Gel formed | No gel formation |

As a result of evaluating gel-forming ability, it can be confirmed that the lipid preconcentrate containing sorbitan laurate, which is a sorbitan saturated fatty acid, exhibited a liquid phase under normal conditions and was converted into a gel state upon contact with a pH 7.4 phosphate buffer solution.

### Preparation Example 6. Preparation of lipid preconcentrate including alopecia and benign prostatic hyperplasia

Dutasteride and finasteride were used as therapeutic agents for benign prostatic hyperplasia.

After the components (amount: g) shown in Table 12 below were sequentially added to a glass vial, the mixture was stirred and mixed using a magnetic stirrer at room temperature to 60 °C. The mixture was then allowed to stand at room temperature for approximately 3 hours to prepare a lipid preconcentrate.

**[Table 12]**

| | Preparation Example 6-1 | Preparation Example 6-2 | Preparation Example 6-3 | Preparation Example 6-4 | Preparation Example 6-5 | Comparative Preparation Example 6-1 | Comparative Preparation Example 6-2 |
|---|---|---|---|---|---|---|---|
| Dutasteride | 5 | 5 | 5 | - | - | 5 | |
| Finasteride | - | - | - | 5 | 5 | - | 5 |
| Phosphatidyl choline | 30 | 35 | 35 | 30 | 35 | 30 | 30 |
| Sorbitan laurate | 20 | 15 | 15 | 20 | 15 | - | - |
| Ascorbic palmitate | - | - | 10 | - | 10 | | |
| Squalene | 20 | 20 | 10 | 20 | 10 | 20 | 20 |
| Cholesterol | 5 | 5 | 5 | 5 | 5 | 5 | 5 |
| Ethanol | 10 | 10 | 10 | 10 | 10 | 10 | 10 |
| DMSO | 10 | 10 | 10 | 10 | 10 | 10 | 10 |
| Result of gel formation | Gel formed | Gel formed | Gel formed | Gel formed | Gel formed | No gel formation | No gel formation |

As a result of evaluating a gel-forming ability, it was confirmed that the lipid preconcentrate containing sorbitan laurate, which is a sorbitan saturated fatty acid, exhibited a liquid phase under normal conditions and was converted into a gel state upon contact with a pH 7.4 phosphate buffer solution.

### Experimental Example 9. Confirmation of sustained-release properties of lipid preconcentrate formation including alopecia and benign prostatic hyperplasia in release test

For the long-term release test (*in vitro* dissolution test) of the lipid preconcentrate, 5 mL of a pH 7.4 phosphate buffer solution containing 0.05% Tween 80 was added to a 6- cc glass vial, and the lipid preconcentrate was injected using a pipette to form a gel. The test was conducted for 28 days at a rotation speed of 30 rpm and a temperature of 37 °C using a rotator (PTR-35, lab23009). The collected samples were analyzed by HPLC under the following conditions.

### <HPLC conditions>

Column: Phenomenox C18 (4.6 x 250 mm, 5.0 µm) or a column having equivalent specifications
Mobile phase: a solution prepared by mixing 100 mL of water and 900 mL of methanol
Flow rate: 1.0 mL/min
Temperature: room temperature
Injection amount: 20 µL
Wavelength: 235 nm

Table 13 shows the results of a long-term release test.

**[Table 13]**

| | Long-term release, day 1 (%) | Long-term release, day 14 (%) | Long-term release, day 21 (%) | Long-term release, day 28 (%) | Long-term release, day 35 (%) |
|---|---|---|---|---|---|
| Preparation Example 6-1 | 2.7 | 54.3 | 74.6 | 90.7 | 100.0 |
| Comparative Preparation Example 6-1 | 10.6 | 100.0 | 100.0 | 100.0 | 100.0 |
| Preparation Example 6-4 | 3.8 | 45.9 | 80.3 | 89.5 | 100.0 |
| Comparative | 12.2 | 100.0 | 100.0 | 100.0 | 100.0 |
| Preparation Example 6-2 | | | | | |

As shown in Table 13, in the cases of Comparative Preparation Examples 6-1 and 6-2, it can be confirmed that 100% release was rapidly achieved on day 14. In contrast, in the cases of Preparation Examples 6-1 and 6-4, it was confirmed that the formulation exhibited a release of 55% or less up to 2 weeks and showed drug release of 85% or more on day 28.

While the present invention has been described in detail above only with respect to the described embodiments, it will be apparent to those skilled in the art that various modifications and variations may be made within the technical spirit and scope of the present invention, and it is understood that such modifications and variations fall within the scope of the appended claims.

### [Industrial Applicability]

The lipid preconcentrate of the present invention can form a liquid crystal during exposure to an aqueous medium, enabling sustained drug release, and can maintain drug release for approximately one week to one month. In addition, the liquid crystal formed by the lipid preconcentrate exhibits sustained-release properties even under low-viscosity conditions, excellent viscoelasticity, and superior ease of use due to low injection force during injection.

## Claims

1. A lipid preconcentrate, comprising:
a phospholipid; a sorbitan saturated fatty acid; and squalene,
wherein a liquid crystal is formed in an aqueous medium.

2. The lipid preconcentrate of claim 1, wherein the content of the phospholipid is 30 to 60 wt% based on the total weight of the lipid preconcentrate.

3. The lipid preconcentrate of claim 1, wherein the sorbitan saturated fatty acid is at least one selected from the group consisting of sorbitan caprylate, sorbitan laurate, sorbitan monolaurate, sorbitan myristate, sorbitan palmitate, sorbitan monopalmitate, sorbitan stearate, sorbitan monostearate, sorbitan tristearate, sorbitan isostearate, and sorbitan monomyristate.

4. The lipid preconcentrate of claim 1, wherein the content of the sorbitan saturated fatty acid is 5 to 45 wt% based on the total weight of the lipid preconcentrate.

5. The lipid preconcentrate of claim 1, wherein the content of the squalene is 2.5 to 30 wt% based on the total weight of the lipid preconcentrate.

6. The lipid preconcentrate of claim 1, further comprising:
a solvent,
wherein the solvent is at least one selected from the group consisting of water, ethanol, isopropanol, propyleneglycol, N-methylpyrrolidone, glycerol, benzylbenzoate, acetic acid, and benzylalcohol.

7. The lipid preconcentrate of claim 1, further comprising:
a drug.

8. The lipid preconcentrate of claim 7, wherein the drug is any one selected from the group consisting of semaglutide, liraglutide, tirzepatide, celecoxib, meloxicam, betamethasone, dexamethasone, methylprednisolone, prednisolone, triamcinolone, dutasteride, and finasteride.

9. A method of preparing the lipid preconcentrate of claim 1, comprising:
preparing a lipid solution by mixing a phospholipid; a sorbitan saturated fatty acid; and squalene.

10. The method of claim 9, wherein the lipid solution further comprises a solvent.

11. The method of claim 9, wherein the lipid solution further comprises a drug.

12. The method of claim 9, further comprising:
preparing a drug solution comprising a drug; and
mixing a lipid solution and the drug solution.
